# EUROPEAN PATENT SPECIFICATION

(11) **EP 2 622 081 B1**
(45) Date of publication and mention of the grant of the patent: **09.03.2016**
(21) Application number: 11761618.5
(22) Date of filing: 28.09.2011
(51) Int. Cl.: A24B 15/20, C12N 9/14, A01H 5/12

(54) **TOBACCO WITH REDUCED CADMIUM CONTENT**
TABAK MIT REDUZIERTEM CADMIUMGEHALT
TABAC À TENEUR RÉDUITE EN CADMIUM

(30) Priority: 30.09.2010 EP 10290518
(43) Date of publication of application: 07.08.2013
(73) Proprietor: Société Nationale d'Exploitation Industrielle des Tabacs et Allumettes S.E.I.T.A., 75014 Paris (FR)
(72) Inventor: JULIO, Emilie, 24240 Cunèges (FR); DORLHAC DE BORNE, Francois, 24680 Lamonzie St. Martin (FR); HERMAND, Victor, 34000 Montpellier (FR)
(74) Representative: UEXKÜLL & STOLBERG
(86) International application number: PCT/EP2011/066882
(87) International publication number: WO 2012/041913

(56) References cited:
- WO-A1-2005/090583
- WO-A1-2009/074325
- WO-A1-2009/074843
- WONG CHONG KUM EDWIN ET AL: "HMA P-type ATPases are the major mechanism for root-to-shoot Cd translocation in Arabidopsis thaliana", NEW PHYTOLOGIST, vol. 181, no. 1, 2009, pages 71-78, XP002624128, ISSN: 0028-646X
- VERRET F ET AL: "Overexpression of AtHMA4 enhances root-to-shoot translocation of zinc and cadmium and plant metal tolerance", FEBS LETTERS, vol. 576, no. 3, 22 October 2004 (2004-10-22), pages 306-312, XP004605705, ELSEVIER, AMSTERDAM, NL ISSN: 0014-5793, DOI: 10.1016/J.FEBSLET.2004.09.023
- MILLS R F ET AL: "The plant P1B-type ATPase AtHMA4 transports Zn and Cd and plays a role in detoxification of transition metals supplied at elevated levels", FEBS LETTERS, vol. 579, no. 3, 31 January 2005 (2005-01-31), pages 783-791, XP004725196, ELSEVIER, AMSTERDAM, NL ISSN: 0014-5793, DOI: 10.1016/J.FEBSLET.2004.12.040
- HANIKENNE MARC ET AL: "Evolution of metal hyperaccumulation required cis-regulatory changes and triplication of HMA4", NATURE (LONDON), vol. 453, no. 7193, May 2008 (2008-05), pages 391-399-METHODS, XP002624129, ISSN: 0028-0836
- HUSSAIN DAWAR ET AL: "P-type ATPase heavy metal transporters with roles in essential zinc homeostasis in Arabidopsis", PLANT CELL, vol. 16, no. 5, 1 May 2004 (2004-05-01), pages 1327-1339, XP002495497, AMERICAN SOCIETY OF PLANT PHYSIOLOGISTS, ROCKVILLE, MD, US ISSN: 1040-4651, DOI: 10.1105/TPC.020487
- MILLS REBECCA F ET AL: "Functional expression of AtHMA4, a P1B-type ATPase of the Zn/Co/Cd/Pb subclass.", PLANT JOURNAL, vol. 35, no. 2, July 2003 (2003-07), pages 164-176, XP002624130, ISSN: 0960-7412

## Description

Heavy metals are naturally present in soil and are taken up by plants to a different degree. Some heavy metals, such as manganese or zinc, are essential for plants, since they represent co-factors required for enzyme activity.

Other heavy metals are however not essential for plants and in some cases a reduction in the heavy metal concentration of plants or parts of plants would be advantageous. Cadmium (Cd) for example is a non-essential heavy metal present in the soil and naturally absorbed by plant roots. Cd is accumulated in leaves, for example in leaves of tobacco plants (Lugon-Moulin *et al.,* 2006). In some places Cd concentrations in soil are increased due to the use of Cd-rich phosphate fertilizers or Cd-contaminated sewage sludge (Karaivazoglou *et al.,* 2007). The mechanisms used by the plants for the uptake, partitioning and accumulation of heavy metals have been described (Verbruggen *et al.,* 2009).

It would be advantageous to reduce the heavy metal content of tobacco plant leaves and different strategies leading to this result have been developed.

For example, a mammalian metallothionein gene under the control of 35S promoter was expressed in tobacco, causing a decrease of 14% of cadmium in field grown plant (Yeargan *et al.,* 1992) or to altered cadmium tissue distribution with a 73 % cadmium reduction in lamina (Dorlhac *et al.,* 1998). More recently, high capacity divalent antiporters, AtCAX2 and AtCAX4, from *Arabidopsis thaliana* have been successfully used to enhance root vacuole Cd sequestration thus obtaining a 15-25% decrease of Cd in lamina of field grown tobacco (Korenkov *et al.,* 2009).

A family of Heavy Metal ATPases (HMA proteins) responsible for distributing heavy metals in the plant tissues after the metals have been taken up by the root have been identified and genes encoding respective proteins have been cloned. Eight types of HMA genes involved in Cd transport to and Cd accumulation in leaves of *Arabidopsis thaliana* are known (Cobbet *et al.,* 2003). In *A*. *thaliana* HMAs from type 1, 5, 6, 7 and 8 are monovalent cation transporters (Cu+ and Ag+; Seigneurin-Berny et *al.,* 2006) and HMAs from type 2, 3 and 4 are bivalent cation transporters (Pb2+, Co2+, Zn2+, Cd2+; Hussain *et al.,* 2004). HMA3 is a vacuolar transporter involved in Cd, Pb and Zn tolerance (Morel et al. 2009).

HMA genes have also been identified in other plants and it has been suggested to generate genetically modified tobacco plants, wherein the expression of a HMA gene is inhibited by RNAi (WO2009/074325).

However, these approaches for reducing heavy metal in tobacco plants are based on the use of genetically modified organisms (GMOs). An approach that is not based on genetically modified plants would have significant advantages.

Mutation breeding has been used for a long time to modify existing traits or to create new valuable traits within a plant cultivar. Recently, this technique has been combined with a high-throughput mutation detection system and has proven to be efficient for the modification of properties in tobacco plants (Julio *et al.,* 2008).

Modern tobacco is an allotetraploid from *Nicotiana sylvestris* (maternal contributor) and *Nicotiana tomentosiformis* (paternal contributor; Yukawa *et al.,* 2006). The tetraploid nature of tobacco (2n=48) complicates the identification of traits in tobacco lines and the preparation of tobacco plants showing completely new traits.

There is thus still a need for tobacco plants with reduced heavy metal content in leaves.

In accordance with the present invention, this problem is now solved by tobacco plants comprising at least one mutation in a HMA gene, wherein the non-mutated HMA gene comprises the nucleotide sequence of SEQ ID NO:1 (nucleotide sequence of HMA 4 gene) or a homolog thereof, wherein the mutation causes a substitution or a deletion or an insertion of at least one amino acid in the polypeptide encoded by the nucleotide sequence and wherein the mutation reduces the heavy metal uptake by the leaves of the plant by at least 30% in relation to the heavy metal uptake of plants comprising SEQ ID NO:1 or the homolog.

The present inventors have surprisingly found that a tobacco HMA 4 gene can be modified such that the HMA 4 protein is inhibited without significant detrimental effects for the plants. In other words, the present invention provides normal tobacco plants that can be used for commercial purposes with significantly reduced heavy metal content in the leaves.

In accordance with the present invention, a homolog of the sequence of SEQ ID NO:1 refers to a sequence with at least 90%, preferably at least 95% sequence identity to the sequence of SEQ ID NO:1. Respective homolog or homologous sequences may represent differences between various tobacco plant lines or sequences derived from a common ancestor. *N. tabacum* is an allotetraploid plant and each plant comprises a genome from *Nicotiana sylvestris* and *Nicotiana tomentosiformis.* As it appears that SEQ ID NO:1 is derived from *N. sylvestris,* the HMA 4 sequence derived from *N. tomentosiformis* represents a homolog of the sequence of SEQ ID NO:1. Similar sequences derived present in different species are also identified in this application as ortholog sequences and represent a specific form of a homolog sequence. The % identity is preferably determined by the BLAST software for determining sequence identity.

In a preferred embodiment, the sequence of SEQ ID NO:1 or of the homolog only contains one or a small number of mutations on the nucleic acid level, for example 1, 2, 3 or 4 nucleotide changes. The mutated sequence thus still has an identity of at least 90%, preferably at least 95% and most preferably at least 98% to the sequence of the HMA 4 gene. The mutation may for example be a miss-sense, a non-sense mutation or a splice mutation.

The tobacco plants of the present invention are preferably *Nicotiana tabacum* plants.

According to one aspect the present invention provides respective tobacco plants, wherein the reduction of heavy metal uptake is determined by growing tobacco plants with and without the mutation under identical conditions on a liquid medium containing the heavy metal and comparing the concentration of the heavy metal in the leaf, stem or shoot of the tobacco plant with the mutation to the concentration of the heavy metal in the leaf, stem or shoot of the tobacco plant that does not have the mutation. It is preferred that the plant and the reference plant are otherwise grown under the same conditions. The concentration of the heavy metal in the leaf, stem or shoot can be identified in amount of heavy metal in relation to amount of plant dry weight material.

The term "reduction in heavy metal uptake" is therefore used in the context of the present invention to describe a reduction in heavy metal concentration in a leaf, stem or shoot of a tobacco plant having a mutation in the HMA 4 gene in comparison to the corresponding tissue of a plant that does not have the mutation but was otherwise grown under the same conditions.

The invention encompasses plants which under these circumstances show a significant reduction in heavy metal concentration in tissues of a tobacco plant. It is particularly preferred that the mutation reduces the uptake of a heavy metal by at least 40%, at least 50%, at least 75% or at least 95%.

In accordance with the present invention, a reduction of uptake can be achieved for one or several but does not have to be achieved for all heavy metals. It is sufficient if a significant reduction is achieved for one heavy metal. It is preferred that the invention provides tobacco plants show a reduced uptake of cadmium, lead or arsenic. In its most preferred embodiment, the present invention provides tobacco plants which have a reduced uptake of cadmium.

Similarly, the reduction of the uptake need not completely eliminate the content of the heavy metal in the plant or plant tissue. In accordance with the present invention it is preferred that the reduction of heavy metal content is in the range of 40% to 70%, 50% to 80%, or 60% to 95%.

Several mutations have been identified in the HMA 4 gene that cause a significant reduction of the heavy metal uptake and are described below. Accordingly, the mutation may represent a miss-sense, a non-sense mutation or a splice mutation.

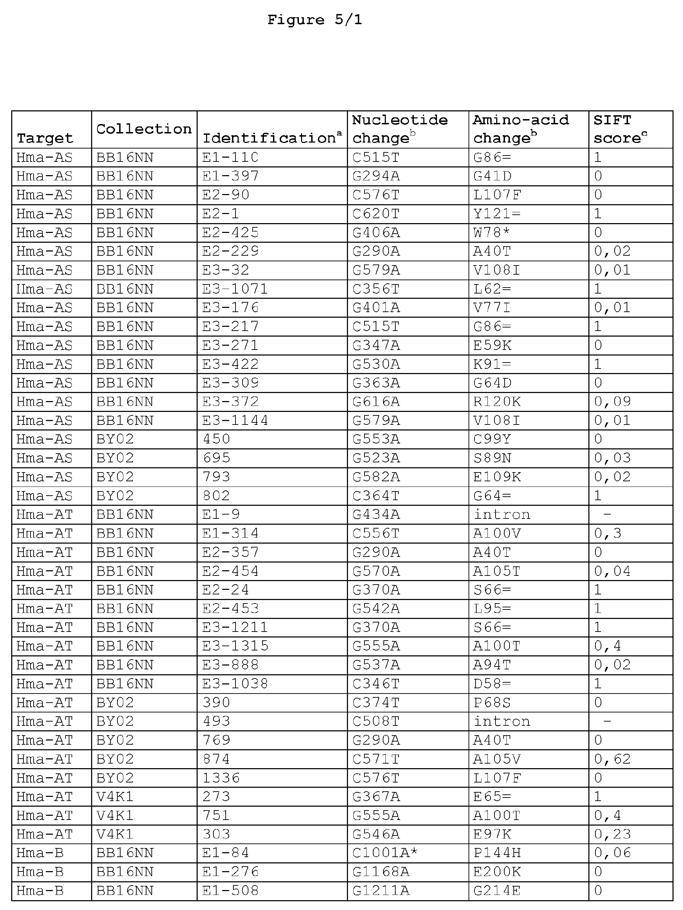

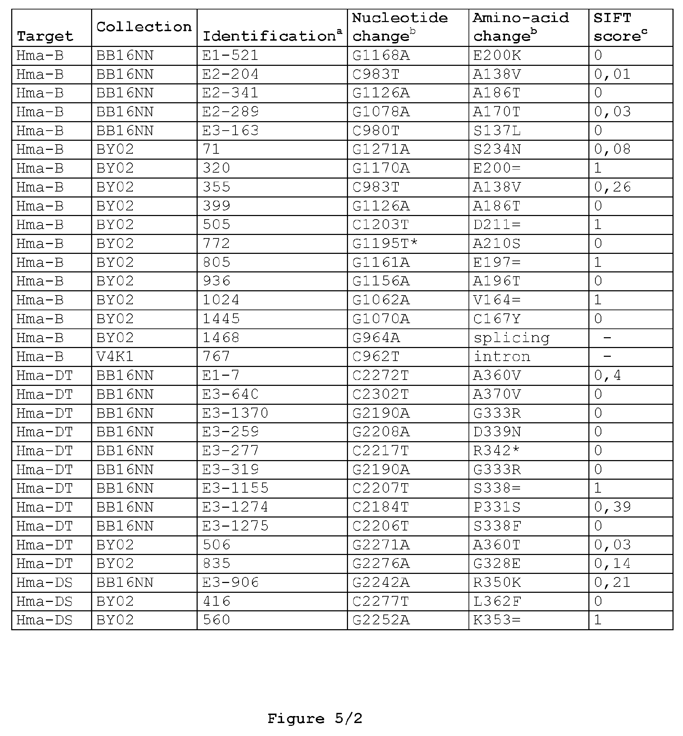

In one embodiment the tobacco plants of the present invention comprise a mutation selected from one of the mutations shown in Figure 5/1 and Figure 5/2. In a preferred embodiment, the tobacco plants comprise one of the mutations shown in Figure 5/1 or 5/2 with a SIFT score of less than 0,05. In the most preferred embodiment the tobacco plants of the present invention comprise a mutation selected from the group comprising the mutations: G294A, C576T, G406A, G347A, G363A, G553A, C374T, G290A, G964A, G1168A, G1211A, G1126A, C980T, G1195T, G1156A, G1070A, C2302T, G2208A, C2217T, G2190A, C2206T or C2277T in SEQ ID NO:1 or the homolog thereof.

According to one embodiment of the present invention, the tobacco plants may comprises a mutation in more than one HMA 4 gene. As indicated, tobacco plants contain HMA 4 genes from *N. Sylvestris* and from *N*. *tomentosiformis.* Plants that are mutated in both HMA 4 genes are also identified as double mutants in the present invention.

The tobacco plants of the present invention can be homozygous or heterozygous for any one mutation in one of the HMA 4 genes. According to a particularly preferred embodiment, plants are provided that contain homozygous mutations in both HMA 4 genes.

The present invention also relates to a part of a tobacco plant as described above, wherein the part is a leaf, a lamina, a cut and/or a cured leaf, root, shoot, stem, flower or seed. Again, for all purposes of the present invention, the part of a *tobacco* plant is preferably a part of a *Nicotiana tabacum* plant. Seed of a tobacco plant as described above, represent a particularly preferred embodiment of the present invention.

The tobacco plants of the present invention or the parts thereof may be used to generate tobacco products well known in the art, including smokeless tobacco products like snus, snuff and cut tobacco, tobacco extract or reconstituted tobacco.

In an alternative embodiment, the tobacco plants of the present invention or the parts thereof are used to generate smoking articles which also represent an embodiment of the invention. Smoking articles are well known and include a cigarette, a small cigar, cigarillo or a cigar or simulated smoking articles containing tobacco.

The plants of the present invention are obtainable by methods comprising the following steps:
(a) screening a library of tobacco plants obtained by mutagenesis for at least one mutation in a HMA gene, wherein the non-mutated HMA gene comprises the nucleotide sequence of SEQ ID NO:1 (nucleotide sequence of HMA 4 gene) or a homolog thereof, wherein the mutation causes a substitution or a deletion or an insertion of at least one amino acid in the polypeptide encoded by the nucleotide sequence and wherein the mutation reduces the heavy metal uptake by the leaves of the tobacco plant by at least 30% in relation to the heavy metal uptake of plants comprising SEQ ID NO:1 or the homolog;
(b) crossing the tobacco plant having a mutation in the HMA gene with a commercial *Nicotiana tabacum* production plant; and
(c) identifying offspring with the mutation in the HMA gene;
(d) repeating steps (b) and (c).

As before, the reduction of heavy metal uptake can be determined by growing tobacco plants with and without the mutation under identical conditions on a liquid medium containing the heavy metal and comparing the concentration of the heavy metal in the leaf, stem or shoot of the tobacco plant with the mutation to the concentration of the heavy metal in the leaf, stem or shoot of the tobacco plant that does not have the mutation.

The methods produce plants, with reduced uptake of the heavy metal by at least 30%, at least 50%, at least 75% or at least 95%.
The plants may have a reduced uptake of one or several heavy metals. It is preferred that the uptake of cadmium, lead or arsenic is reduced.
According to a preferred embodiment, steps (a) and/or (c) of the method use an assay analyzing the nucleotide sequence of the tobacco (*Nicotiana tabacum)* plant. Respective assays for nucleotide analysis are well known in the art of molecular biology and include PCR-based techniques, DNA sequencing, hybridization and/or RFLP techniques.

A tobacco product or a smoking article can be obtained from the plants of the present invention by harvesting the leaves, stems and/or shoots and producing a tobacco product or smoking article from the same.

### BRIEF DESCRIPTION OF THE FIGURES

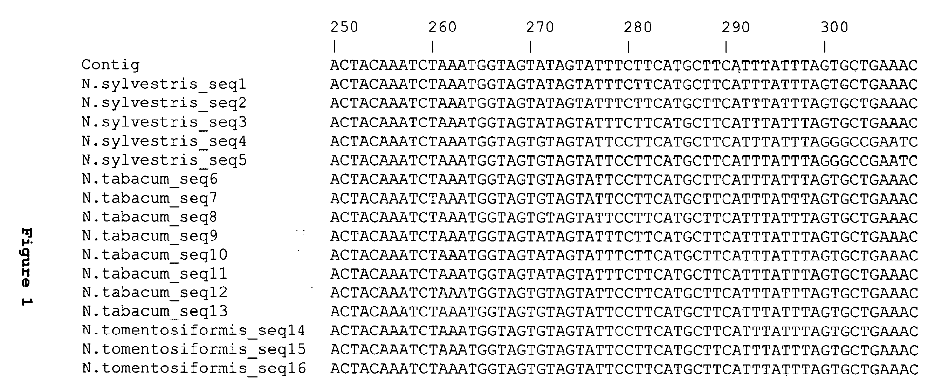

- **Figure 1:**: Partial sequence alignment of part A amplification in *N. tabacum, N. sylvestris* and *N. tomentosiformis.*
The first sequence is the reference contig. In this alignment three different sequences are present: sequences 1, 2, 3, 9, 10, 11 are shared between *N. tabacum* and *N. sylvestris.* Sequences 6, 7, 8, 12, 13, 14, 15, 16 are shared between *N. tabacum* and *N. tomentosiformis.* Sequences 4 and 5 are specific to *N. sylvestris.*

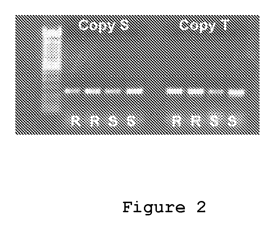

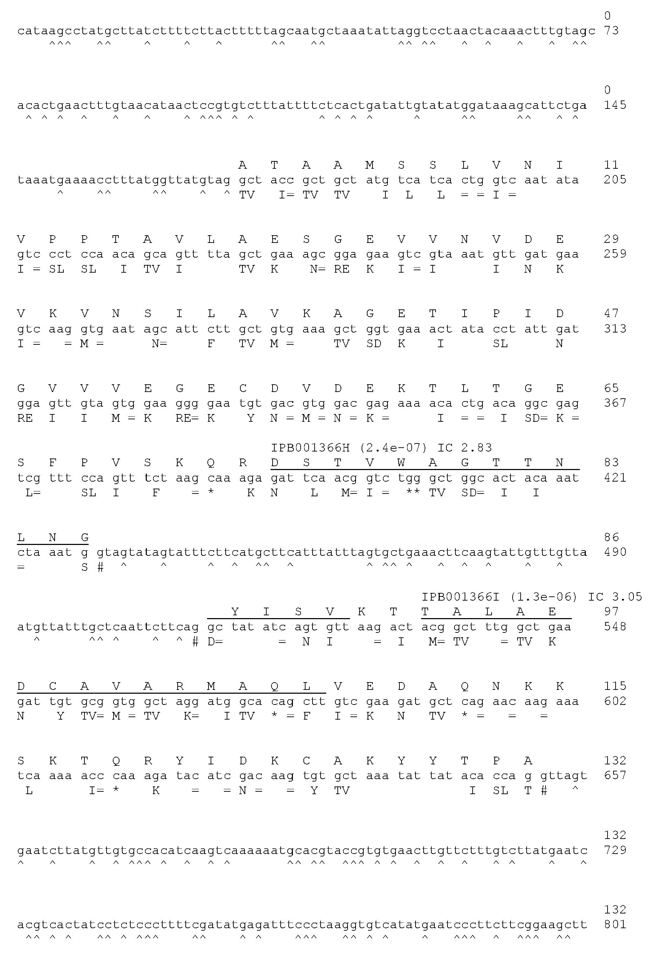

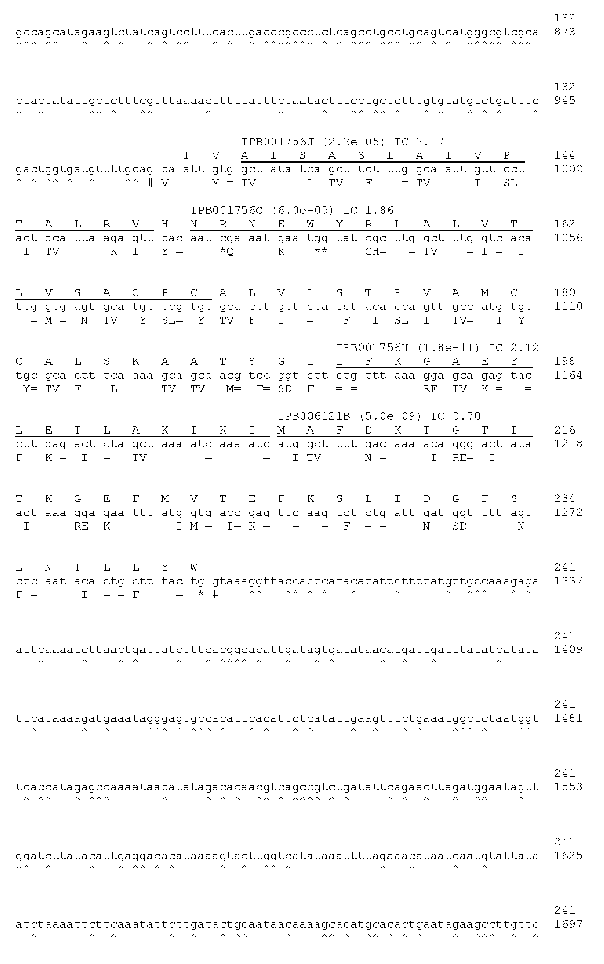

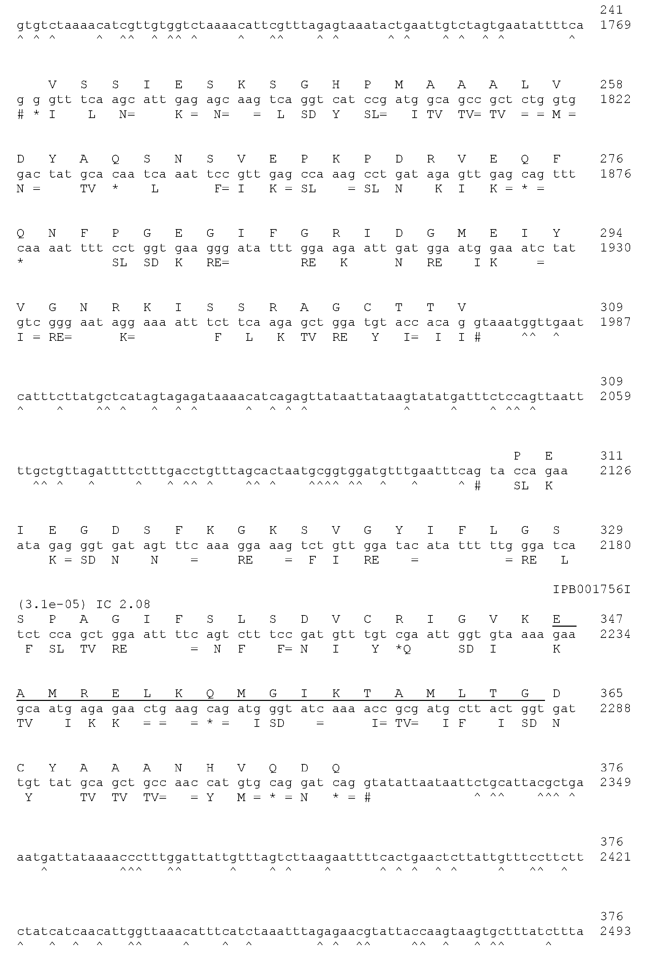

- **Figure 2:**: ds cDNA amplification of copies 1 and 2 of the HMA orthologs in *N. tabacum.*
Specific primers designed to anneal in exonic regions of part A were used to test the expression of the two copies in roots (R) and shoots (S). HmaAS-F/HmaA-RT-R primers pair amplify the *N. sylvestris* origin sequence (Copy *S*) and HmaAT-F/HmaA-RT-R primers pair amplify the *N. tomentosiformis* origin sequence (Copy T). Both copies are expressed in *N. tabacum.*
- **Figure 3:**: CODDLE analysis of the whole contig sequence (SEQ ID NO:1).
First line: amino-acid sequence of the HMA4 protein
Second line: DNA sequence of the HMA4 gene Third line: below each nucleotide is an indication of the changes that can be detected with EMS mutagenesis.
- **Figure 4:**: CE-SSCP profile obtained by PCR amplification of the DNA mutant collection with primers Hma4-BF/Hma4-BR. The HmaA4-BF primer is labeled with FAM fluorophore, and the DNA strand appears in blue. The Hma-BR primer is labeled with VIC fluorophore, and the DNA strand appears in green.
1: DNA profile of a wild type DNA
2: DNA profile of a mutant DNA (family E1-276 of the collection), characterized by an additional peak corresponding to the mutated DNA strand (red arrow).
- **Figure 5:**: Table summarizing the mutations found in HMA targets after cloning and sequencing.
^{a} = Identification number of the mutant family as described in the collection.
^{b} = nucleotide or amino-acid change and position of the mutation as described with CODDLe analysis on contig 1 (original nucleotide/amino-acid+ position +new nucleotide/amino-acid).
"=" represents silent mutation, "*" represents stop codon.
^{c} = SIFT score was obtained with the bioinformatics program SIFT (Sorting Intolerant from Tolerant) on contig 1. SIFT scores < 0.05 are predicted to be deleterious to the protein.
* non EMS mutation (G/C to A/T)

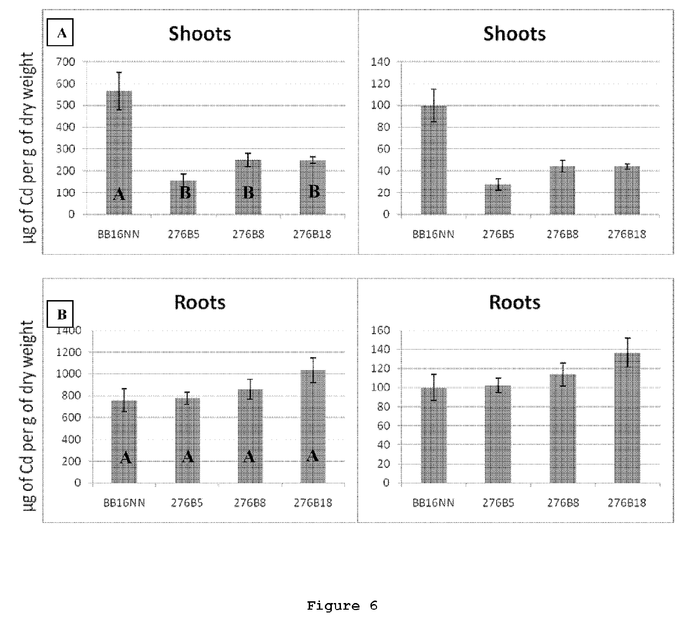

- **Figure 6:**: Cadmium content in three M3 E1-276 mutant lines (276B5; 276B8, 278B18) compared to the wild type (BB16NN).
Cadmium was measured in shoots (A) or roots (B). On the left, graphs represent cadmium accumulation in µg per g of dry weights. On the right, graphs represent cadmium accumulation normalized in mutants with cadmium accumulation in wild type.
Cadmium content in shoots: the letters "A" in "B" in bars represent two distinct groups in a student "T" test performed on these samples.
Cadmium accumulation in roots: the letter "A" in bars indicates that all the means in this graph belong to the same statistical group.
Error bars represent standard deviation.

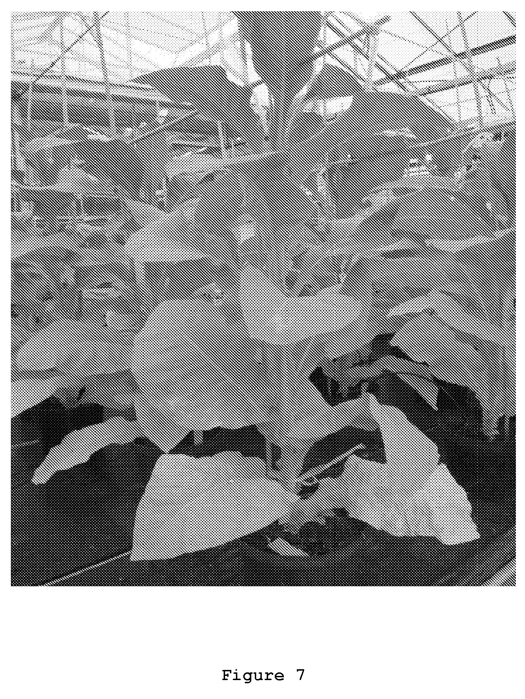

- **Figure 7:**: Picture of a F1 cross between the mutant E1-276-B18 and an industrial flue-cured tobacco plant showing the viability of the mutants.

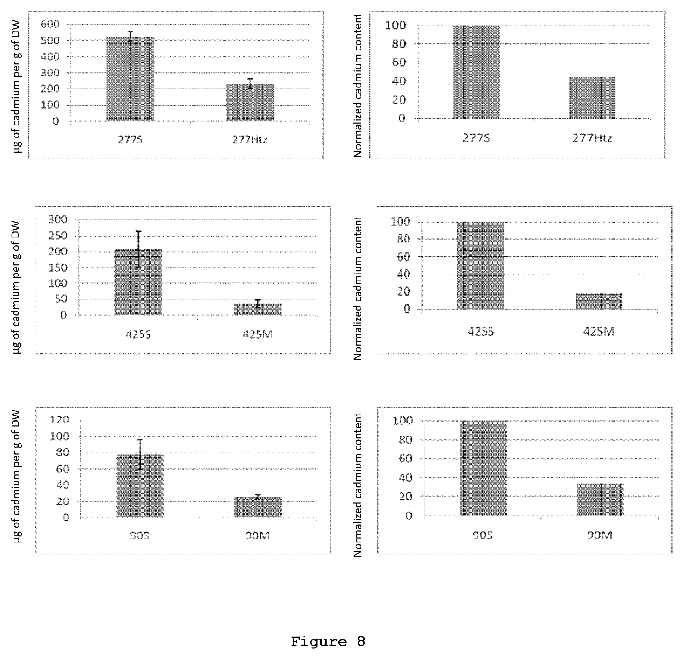

- **Figure 8:**: Cadmium accumulation in shoots of EMS lines (M mutated plants; Htz heterozygous mutant plants; S wild-type plants; DW dry weight). The graphs on the left represent the amount of cadmium accumulated in the shoots. The error bars reflect the standard error. On the right side, the accumulation of cadmium in the mutated line is represented as a percentage with respect to its wild-type.

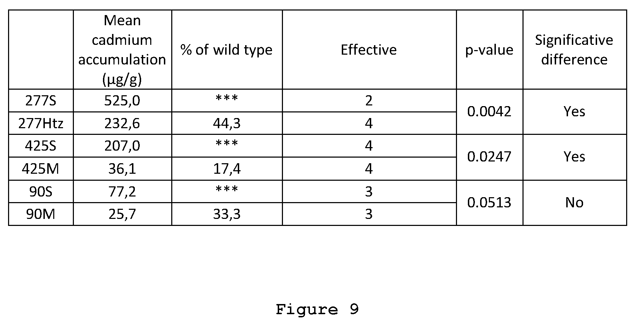

- **Figure 9:**: Statistal analysis of EMS lines. The p-values presented in this chart are derived from the student's t-test (http://www.graphpad.com). The differences observed between each mutated line and its wildtype is statistically relevant when the p-value is lower than 0,05 (95% confidence).

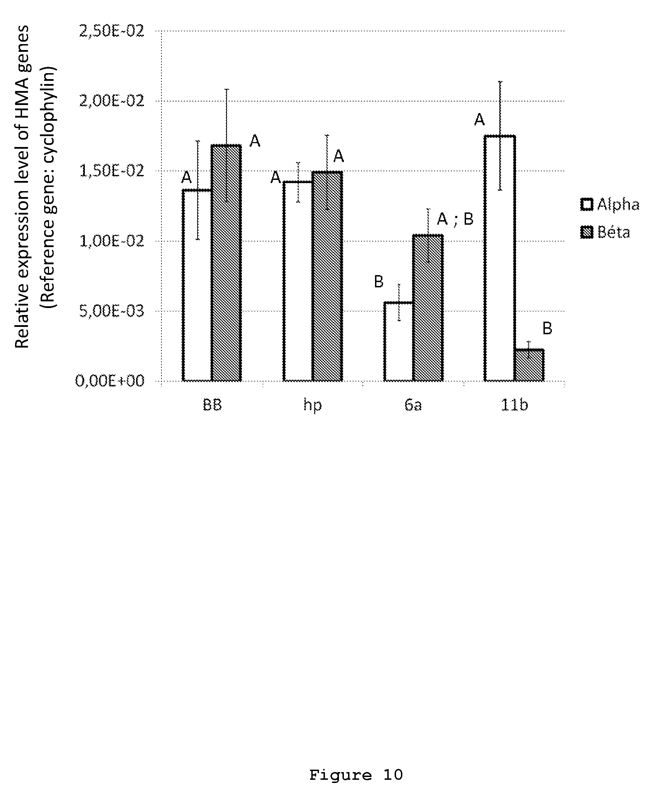

- **Figure 10:**: Accumulation of RNA transcripts in RNAi lines. The bars represents the mean of the accumulation of transcripts of either HMA alpha or HMA beta relative to the transcript accumulation of the reference gene cyclophilin and L2. Three plants were used per line. The error bars represent standard error. The letters A and B represent the two groups found by student's t-test realized with the open software "R".

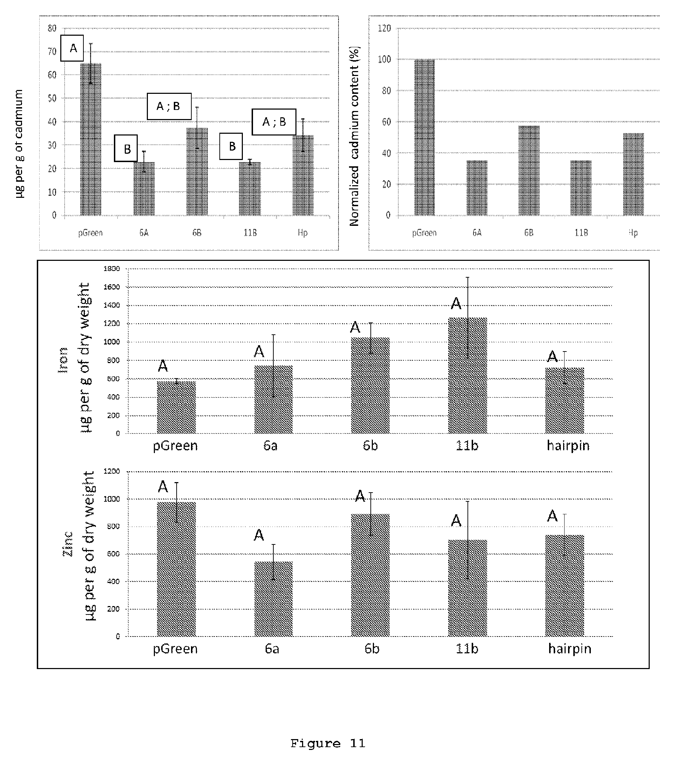

- **Figure 11:**: Metal accumulation in shoots of RNAi lines. For each line, 4 plants were analyzed (only 3 for pGreen). The cadmium accumulation is represented at the top. On the left, the cadmium accumulation is expressed as a mean. On the top right, those means are expressed as percent of wild-type. At the bottom, the accumulation of iron and zinc in shoots is represented. The error bars represent standard error. The letters A and B represent statistical groups realized with the open software "R". The letters are independent in each graph.

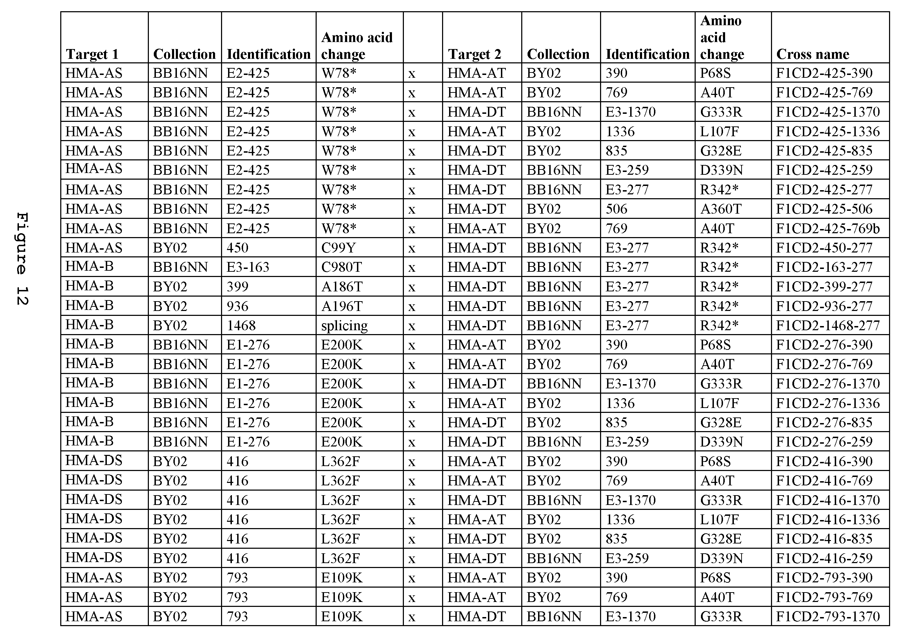

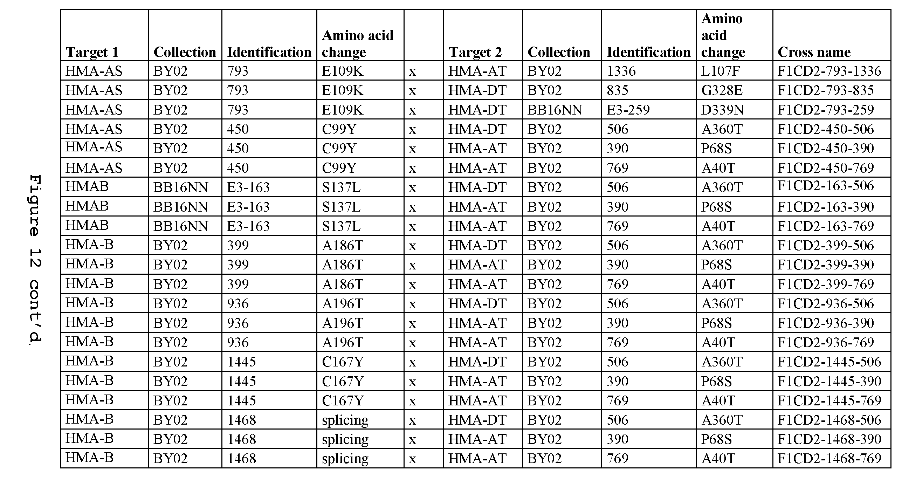

- **Figure 12:**: Crosses performed between tobacco mutants to accumulate both mutated copies in one genome. Mutants were first backcrossed with wild-type and then crossed between them to obtain F1 generation. The F1 name is described as F1CD2, with the following numbers representing both mutant identifications.

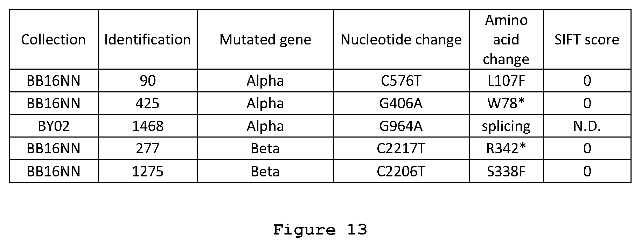

- **Figure 13:**: EMS lines. The lines are presented with the collection from which they originate and the mutation that affects either one gene or the other.

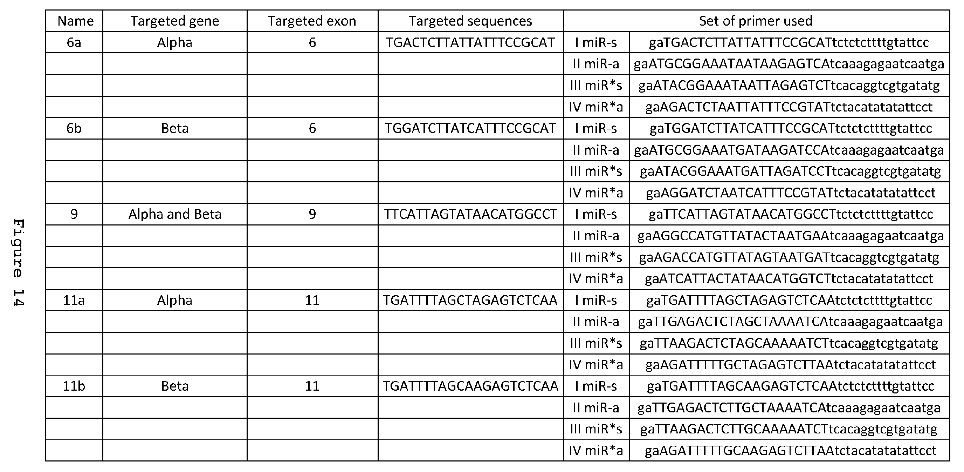

- **Figure 14:**: amiRNA constructs. All of the targeted sequences and their corresponding primer sequences were obtained using online software (http://www.weigelworld.org/).

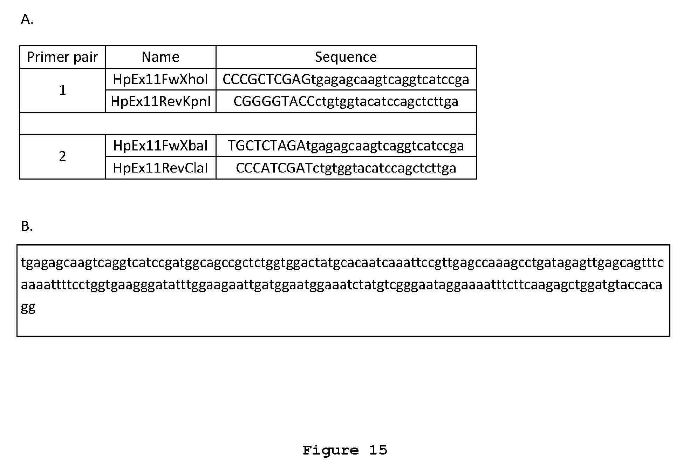

- **Figure 15:**: Targeted sequence of the hairpin construct. The primer pairs (A) were designed to amplify the same portion of *HMA* gene (B) but they carry different restriction sites.

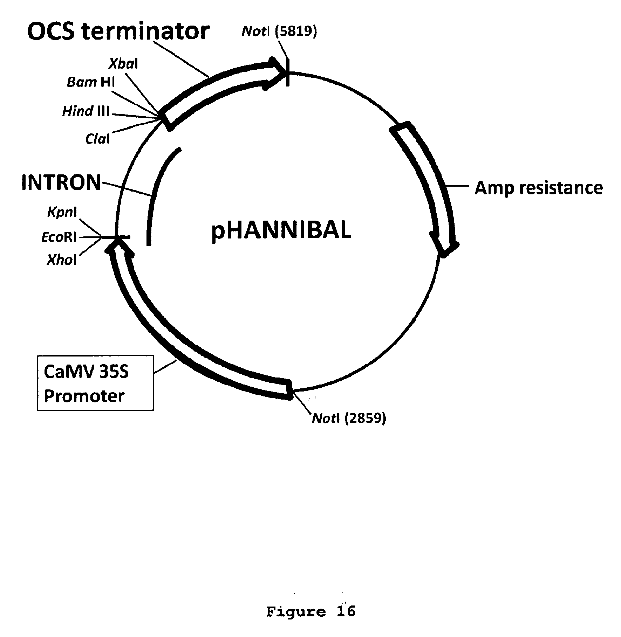

- **Figure 16:**: pHannibal vector. This vector contains 2 sets of restriction sites separated by an intron.

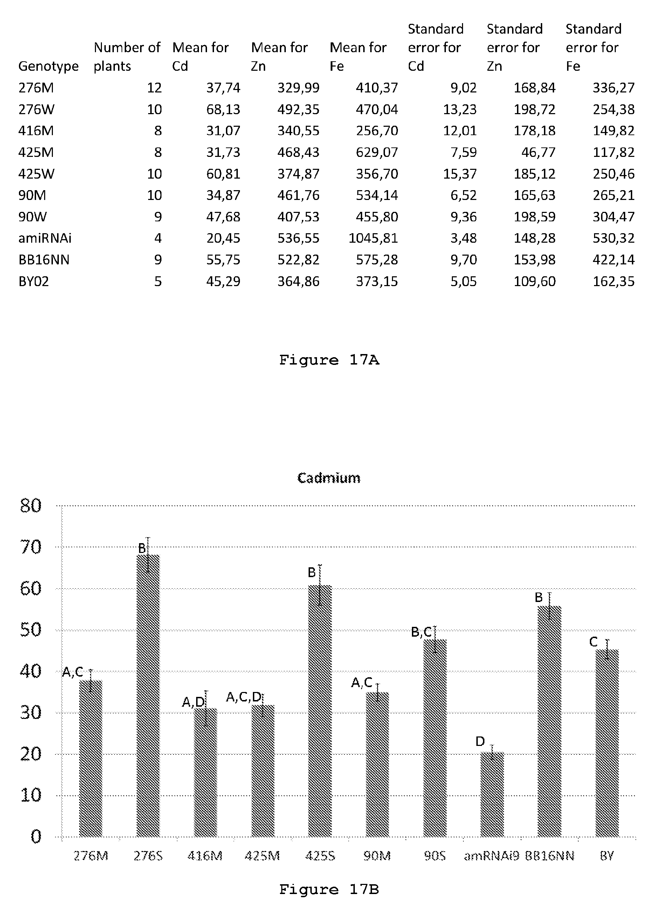

- **Figure 17:**: Metal accumulation in shoots; graphical results obtained by Tukey multiple comparisons of means. The error bars represent standard error. The letters A, B, C and D represent statistical groups realized with the open software "R" for cadmium analyses.

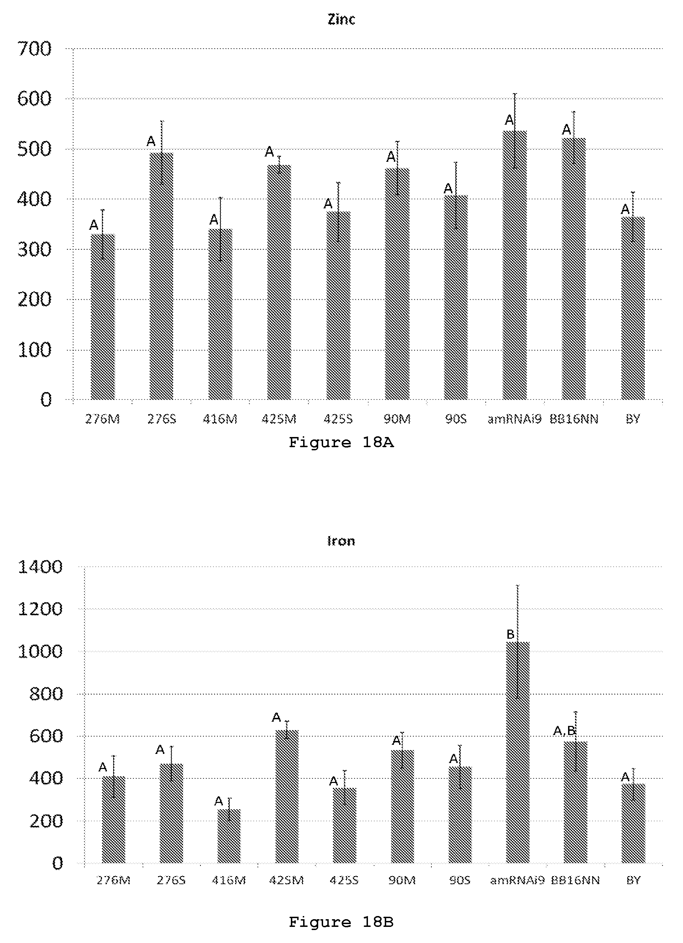

- **Figure 18:**: Metal accumulation in shoots; graphical results are obtained by Tukey multiple comparisons of means.

### EXAMPLE 1

### Tobacco mutant collection

### Plant material

Seeds of three tobacco lines BB16NN (Delon et al. 1999; Institut du Tabac de Bergerac, Accession N°1139), BY02 and V4K1, were used for creating *Nicotiana tabacum* mutant libraries. BB16NN and BY02 are burley type tobaccos, and V4K1 is a flue-cured type tobacco.

For copy number assessment, comparison of sequences with ancestors was done with tobacco lines ITB 645 (*Nicotiana tomentosiformis*) and ITB 626 (*Nicotiana sylvestris*)*.*

### EMS mutagenesis

The mutant collection has been described in Julio *et al.,* 2008. In short, two tobacco EMS (ethylmethane sulfonate) mutant libraries termed L1 and L2 were constructed by soaking tobacco BB16NN seeds (6000 seeds per library) overnight (16h) in 0.8% EMS (L1) or 0.6% EMS (L2) solutions, followed by 12 washings of 30 min in water under shaking. In addition, L1 library seeds were pre-germinated for 2 days before EMS treatment.

Two further collections were developed from BY02 (termed L3) and V4K1 (termed L4) seeds with a 0.7% EMS treatment and without pre-germination.

The mutagenized M1 seeds were grown to M1 plantlets in a greenhouse and transferred to the field to give M2 generation by self-pollination. M2 seeds were collected from each M1 plant and stored until use. Leaf material was collected from 8 M2 seeds sown in a single pot in greenhouse and pooled (two 8 mm diameter discs for each plant i.e. ∼100 mg fresh weight per family) to constitute pooled M2 family. DNA was extracted from the leaf material using QIAGEN Dneasy 96 Plant Kit according to manufacturer's instructions.

### EXAMPLE 2

### Copy Number Assessment

### PCR amplification

Part of the genomic sequence of an HMA 4 of *Nicotiana tabacum* gene is shown in SEQ ID NO:1.

*N. tabacum* is an allotetraploid plant comprising a genome from *Nicotiana sylvestris* and *Nicotiana tomentosiformis.* It appears that SEQ ID NO:1 is derived from *N. sylvestris.*

Four genomic regions of this HMA 4 gene, identified as part A, B, C and D, were amplified in *N. tabacum, N. sylvestris* and *N*. *tomentosiformis* by using the respective primer pairs:
PartA-F (CTACCGCTGCTATGTCATCAC) SEQ ID NO:2 and
   PartA-R (TAGCACACTTGTCGATGTATC) SEQ ID NO:3;
PartB-F (GATACATCGACAAGTGTGCTA) SEQ ID NO:4 and
   PartB-R (CTCCTTTAGTTATAGTCCCTG) SEQ ID NO:5;
PartC-F (AGTAAATACTGAATTGTCTAGTG) SEQ ID NO:6 and
   PartC-R (GATGTTTTATCTCTACTATGAGC) SEQ ID NO:7;
PartD-F (GACCTGTTTAGCACTAATGCG) SEQ ID NO:8 and
   PartD-R (TTATAATCATTTCAGCGTAATGCAG) SEQ ID NO:9.

PCR amplifications were carried out in a 20 µl volume containing 1 µl DNA, 10X AmpliTaq buffer (Applied Biosystems, Foster City, USA), 1 µl dNTPs (Applied Biosystems, 2.5 mM each), 50 ng of each primer and 0.05 U AmpliTaq Polymerase (Applied Biosystems). PCR was conducted using a thermal cycler (GeneAmp® PCR System 2700, Applied Biosystems) as follows: 35 cycles of 94°C for 30s, 62°C for 45s, 72°C for 1 min, followed by 7 min at 72°C for final extension.

### Results

Four parts of the contig sequence shown in SEQ ID NO: 1 were amplified in *N. tabacum, N. tomentosiformis* and *N. sylvestris.* Part A corresponds approximately to exons 4 to 5, part B corresponds approximately to exon 6, part C corresponds to exon 7 and part D corresponds to exon 8. Six to ten clones were sequenced for each PCR product. An example of sequence alignment in part A is shown in Figure 1. Some sequences were common between *N*. *tabacum* and its ancestors (*N. sylvestris* and *N*. *tomentosiformis*); others are specific to one ancestor. Results are summarized in Table 1, below.

**Table 1: Results obtained after cloning and sequencing PCR products amplified with primers HmaA-F/R (partA), HmaB-F/R (partB), HmaC-F/R (partC) and HmaD-F/R (partD).**

| | Part A | | Part B | | Part C | | Part D | |
|---|---|---|---|---|---|---|---|---|
| | Specific | Common with N.tabacum | Specific | Common with N.tabacum | Specific | Common with N.tabacum | Specific | Common with N.tabacum |
| N. sylvestris | 2 | 1 | n.f. | 1 | n.f. | 1 | n.f | 1 |
| N. tomentosiformis | n.f. | 1 | 1 | n.f. | 1 | n.f. | n.f. | 1 |

| | | | | | | | | |
|---|---|---|---|---|---|---|---|---|
| As 6 to 10 clones were sequenced per PCR product, results are expressed as a minimal number of existing copies. n.f. = not found | | | | | | | | |

These results show that *Nicotiana tabacum* contains at least two HMA4 loci, one locus from each of the two ancestors. As a consequence, any *Nicotiana tabacum* plant may contain at least four different HMA 4 alleles.

### RNA extraction and ds cDNA testing

RNA was extracted from roots and shoot of two months old plants with RNeasy Plant Kit according to manufacturer's instructions (Qiagen). cDNA and ds cDNA was prepared according to manufacturer's instructions by using the MINT cDNA synthesis kit from Evrogen.

Expression of both loci was assayed by amplifying ds cDNA coming from *N. tabacum* roots and shoots with specific primers designed on the basis of part A exonic regions. HmaAS-F/HmaA-RT-R was used to amplify the *N. sylvestris* origin sequence and HmaAT-F/HMA-RT-R for the *N. tomentosiformis* origin sequence. Fragments were amplified for both copies (*N. sylvestris* and *N*. *tomentosiformis*) of the gene, as demonstrated in Figure 2, supporting the conclusion that the sequences present at both loci are expressed in *N. tabacum* roots and shoots.

Amplification of the two copies in expressed sequences was tested in region A, with specific non-labeled primers pairs:
- forward primers Hma-AS-F GGTGAATAGCATTCTTGCTGTG SEQ ID NO:19; and HmaAT-F, GTTGAATAGCATTCTTGCTGTT SEQ ID NO:21; and
- a new common reverse primer HmaA-RT-R, CTTGTTCTGAGCATCTTCGAC SEQ ID NO:20, designed to bind in the exonic region.

PCR was carried out in the same conditions than for copy number assessment. PCR products were visualized on a 1.5% agarose gel with EtBr (ethidium bromide) under UV.

### EXAMPLE 3

### Mutant Screening via PCR

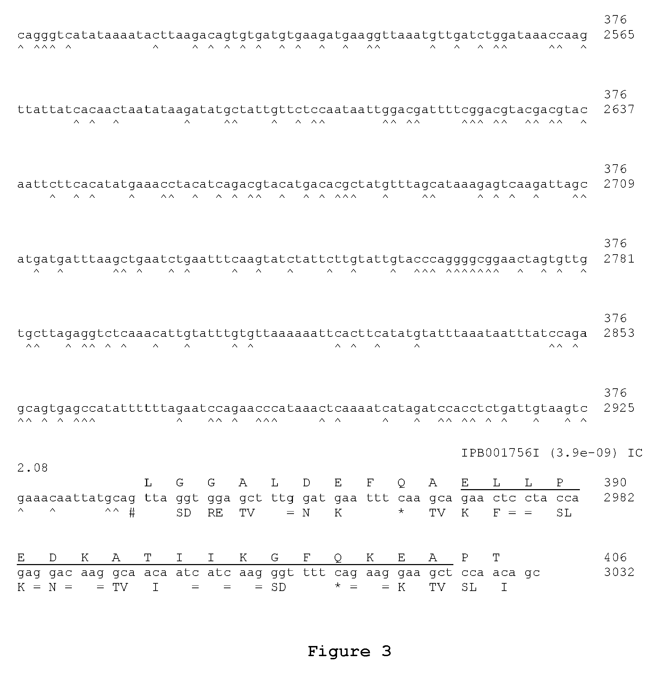

New primers were designed to amplify specifically the regions to be used for mutant screening. Primers were selected according to three criteria:
- Positions of intron and exon: exonic regions are preferred, even if primers can be designed in intronic regions.
- High impact of EMS mutations on the protein sequence: the impact of the mutation on the protein function was assessed by the CODDLe software [Choosing codons to Optimize Discovery of Deleterious LEsions]. CODDLe identifies the region(s) of a user-selected gene and of its coding sequence [CDS] where the anticipated point mutations are most likely to result in deleterious effects on the gene's function (Till *et al.,* 2003). CODDLe results on the whole contig sequence is shown in Figure 3.
- Length of the sequence: the maximum length of the sequence for CE-SSCP screening is 500 bp. A large sequence is preferred to maximize the chance to discover mutants.

The following new primer pairs were designed to amplify:
Region A: HmaAS-F/HmaA-R primer pair amplifies the *N. sylvestris* origin sequence and HmaAT-F/HMA-R amplifies the *N. tomentosiformis* origin sequence.
Region B: HmaB-F/HmaB-R primer pair.
Region D: HmaDS-F/HmaDS-R primer pair amplifies the *N. sylvestris* origin sequence and HmaDT-F/HmaDT-R primer pair amplifies the *N. tomentosiformis* origin sequence.

The specificity of primer pairs was checked by cloning and sequencing ten PCR products.

Fluorescent labeled primers (6-FAM (blue), VIC (green), NED (yellow); all from Applied Biosystems) were used for Capillary Electrophoresis-Single Strand Conformation Polymorphism analysis (CE-SSCP). PCR reactions were carried out in the same conditions as used for copy number assessment.

Two different 410 bp copies were amplified in regions A with:
- forward primer HmaAS-F (VIC-GGTGAATAGCATTCTTGCTGTG) SEQ ID NO:10; or
- forward primer HmaAT-F (NED-GTTGAATAGCATTCTTGCTGTT) SEQ ID NO:12; and
- common reverse primer HmaA-R (6FAM-GCACAACATAAGATTCACTAAC) SEQ ID NO:11.

A unique 386 bp sequence was amplified in region B, with:
- forward primer HmaB-F 6FAM-GTCTGATTTCGACTGGTGATG SEQ ID NO:13; and
- reverse primer HmaB-R VIC-AAGAATATGTATGAGTGGTAACC SEQ ID NO:14.

Two 283 bp copies were amplified separately in region D, with:
- primer pair HmaDT-F, 6FAM-GAAATAGAGGGTGATAGTTTCC SEQ ID NO:15, and HmaDT-R, NED-CATTTCAGCGTAATGCAGAATTT SEQ ID NO:16, for the first copy; and
- HmaDS-F, VIC-GAAATAGAGGGTGATAGTTTCA SEQ ID NO:17, and HmaDS-R, 6FAM-CATTTCAGCGTAATGCAGAATTA SEQ ID NO:18, for the other copy.

### Capillary electrophoresis

Fluorescent-labeled PCR products were diluted 1/20 in water before CE-SSCP analysis. Prior to loading on ABI Prism® 3130 (Applied Biosystems, Foster City, USA), 1µl of diluted sample was added to 10 µl formamide (Applied Biosystems) and 0.1 µl Genescan-500 LIZ Size Standard (Applied Biosystems). A denaturation step of 94°C for 3 min followed by cooling on ice was used for single strand conformation analysis.

Running conditions on ABI Prism® 3130 were as follows: 36 cm capillary array (16 capillaries), run temperature of 22°C, sample injection of 1 kV for 15 s and separation of 15 kV for 40 min. The non denaturing separation medium was POP Conformational Analysis Polymer (Applied Biosystems) 5%, glycerol (Sigma-Aldrich) 10% in 1X Buffer (10X) with EDTA (Applied Biosystems). The running buffer was glycerol 10% in 1X Buffer (10X) with EDTA. Results were analyzed with GeneMapper 4.0 (Applied Biosystems) software.

### Cloning and sequencing

PCR products were cloned into pGEM-T vector Systems (Promega, Madison, USA) and transformed into *E. coli* according to manufacturer's instructions. Ten clones were sequenced for each family, using BigDye Terminator Sequencing Kit v3.1 (Applied Biosystems) and ABI Prism® 3130 (Applied Biosystems). Nucleotic sequences were aligned with Multalin (http://npsa-pbil.ibcp.fr/NPSA/npsa multalinan.html).

### Results

### Mutant screening in the HMA4 orthologs in tobacco

Primers were fluorescently labeled and were used to amplify the DNA of the mutant collection. Fluorescent-labeled PCR products were analyzed by CE-SSCP as described above.

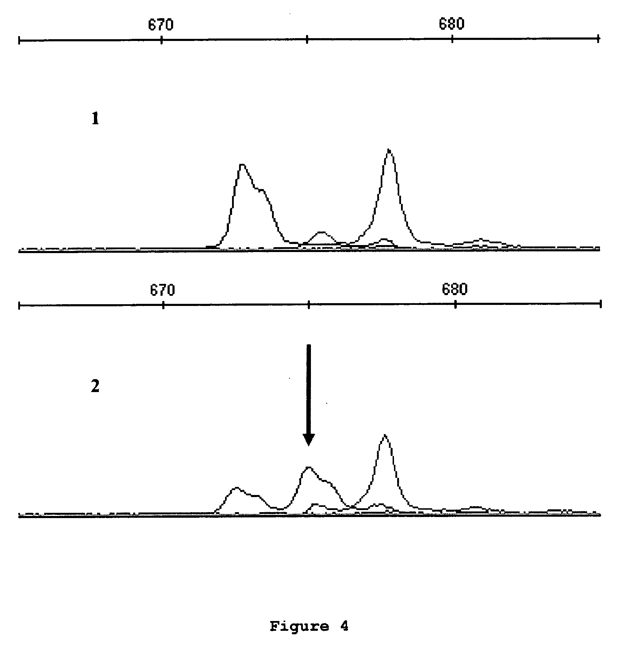

Mutants were detected by the presence of additional peaks on the analysis profile, compared to the control (DNA of a non mutated tobacco). An example with primers Hma4-BF/Hma4-BR is shown in Figure 4.

PCR products of mutants were cloned and sequenced to characterize the position of the mutation. Sequences obtained were aligned and compared to the control. Impact of mutations on protein function was analyzed by the Sorting Intolerant From Tolerant (SIFT) program (Ng and Henikoff 2003).

Complete results of mutations obtained in Hma-AS, Hma-AT, Hma-B, Hma-DS and Hma-DT are summarized in the Table shown in Figure 5/1 and 5/2.

Nineteen mutations were obtained for Hma-AS target, 18 for Hma-AT, 20 for Hma-B, 11 for Hma-DT and 3 for Hma-DS.

Non-sense mutations could be obtained for Hma-AS and Hma-DT. Silent and miss-sense mutations represent respectively 9% to 33 % and 61% to 81% of the total number of mutations, as presented in table 2. One mutation in a splicing region could be found in Hma-B target. Mutations affecting the same amino-acid could be found. Two exactly redundant mutations were found in Hma-AT, one in Hma-AS, 3 in Hma-B and one in Hma-DT. Of the 71 mutations obtained, 2 transversions were observed (2.8%) (instead of G/C to T/A transitions expected with EMS treatment.)

**Table 2: Percentage of mutation according to the target and the type of mutation.**

| | **Total** | **Silent** | **Miss-sense** | **Non-sense** | **Intron** | **Splicing** |
|---|---|---|---|---|---|---|
| **Hma-AS** | 19 | 31,6 | 63,2 | 5,3 | 0,0 | 0,0 |
| **Hma-AT** | 21 | 22,2 | 57,1 | 0,0 | 9,5 | 0,0 |
| **Hma-B** | 20 | 20,0 | 70,0 | 0,0 | 5,0 | 5,0 |
| **Hma-DT** | 11 | 9,1 | 81,8 | 9,1 | 0,0 | 0,0 |
| **Hma-DS** | 3 | 33,3 | 66,7 | 0,0 | 0,0 | 0,0 |

### EXAMPLE 4

### Heavy Metals Translocation

### Hydroponic culture

Selected M2 EMS lines were grown in soil in a greenhouse in order to obtain homozygous mutant seeds. DNA was extracted from 2 month old plants for genotyping by CE-SSCP. Homozygous mutant lines were self-pollinated to obtain homozygous mutant seeds (M3). M3 seeds were germinated on Whatman paper soaked with a Hoagland-derived solution (KNO3 2,5mM ; NaH2PO 4 0,5 mM ; Ca(NO3)2 2,5mM ; MgSO4 0,5 mM ; FeNaEDTA 0,1 mM ; H3BO3 50 µM ; MnSO4 50 µM ; ZnSO4 15µM ; MoO4Na2 3µM ; KI 2,5µM ; CuSO4 50 µM ; CoCl2 44 µM). After 3 weeks, plants were transferred to the Hoagland-derived solution media were there growth is prolonged for 2 weeks. The solution media was then complemented with a final concentration of 10µM of CdCl2. After one week of treatments plants were cut in two parts: roots and shoots and were harvested independently.

### Greenhouse testing

M2 lines were sown on Whatman paper until germination and 30 mutants plantlets were transferred in floating trays (20cm x 30cm), with five control plants randomly placed in the tray (non-mutagenized original lines). DNA was extracted from 1 month old plants for CE-SSCP genotyping to characterize homozygous, heterozygous and wild type plants for the HMA mutation. After one month and a half, the solution media was complemented with a final concentration of 1µM to 5µM of CdCl2. After one week of treatments, roots were rinsed with CaCl2 (1 mM) and plants were cut in three parts: roots, stem and shoots. Samples were pooled according to their genotype, with four plants per genotype.

### Cadmium detection by ICP-MS

Samples were dried at 80°C for 72 hours and dry weight was measured. Metal contents were extracted in chloridric acid (1 mM) for 1 hour at 75°C.

Metals content in the chloridric acid were dosed by Inductively Coupled Plasma Mass Spectroscopy (ICP-MS) from Agilent (7500cx series). Ions are analyzed based on their mass to charge ratios (Newman, 1996). This technique has already been used for analysis of trace elements in many fields (e.g. Kelly *et al.,* 2002) and permit to detect cadmium traces as low as 10 ppt.

To obtain cadmium content the concentration of the dosed solution is multiplied by the dilution factor (this gives the amount of metal in the sample) and divided by the mass of the sample. To obtain translocation, the amount of metal in shoots is divided by the amount of metal in the whole plant (metal in roots plus metal in shoots).

### Statistical analysis

For each line, mean value and standard deviation was calculated. The statistics software "R" was used to perform a student "T" test to compare those means (http://www.r-project.org/).

### EXAMPLE 5

### Backcrosses of mutation into elite lines

Mutants containing an interesting mutation were backcrossed with the original non-mutagenized plant in order to remove additional mutations present in the rest of the genome, not related with cadmium transfer. An example of a F1 cross of an industrial Flue-cured tobacco and E1-276-B18 mutant is shown in Figure 7. Mutants from the collection and elite lines were grown in greenhouse in floating beds. DNA of 30 plants per mutant family was extracted and analysed by CE-SSCP. Heterozygous plants were transferred in 5 litter's pots, along with elite lines. At flowering time, pollen of mutant was transferred on flower of the original line (BB16NN, BY02 or V4K1), cleared out of its stamen. Several cycles of backcrosses can be performed (BC1, BC2...) before fixing the mutation by two cycles of self-pollination of backcrossed mutant plants (BCxS1 and BCxS2).

### EXAMPLE 6

### Heavy Metals Translocation

### Hydroponic culture

### A. Mutation E200K

Experiments were conducted with the mutation E200K, in region B of the sequence, in the E1-276 line of BB16NN collection. Homozygous M2 mutant lines were self-pollinated to obtain homozygous mutant seeds (M3). Experiment was conducted on young M3 plantlets.

### Results

Cadmium content in the homozygous M3 E1-276 mutant line is significantly reduced. Figure 6 describes the results obtained in terms of cadmium accumulation in roots and in shoots. In shoots cadmium content is reduced to different degrees and can be reduced by more than 50% in the lines of the present invention in comparison to wild type. As can be seen in Figure 6, cadmium content is reduced in shoots for example for more than 60% and in some plants even for more than 70%.

### B. Mutation E3-277

Further experiments regarding the uptake of Cd and Zn were conducted with plants homozygous for the mutation E3-277.

### Results

The results are shown in the Table 3, below and show that cadmium content in the homozygous E3-277 plants are significantly reduced, whereas the content of Zn is slightly reduced in comparison to control plants.

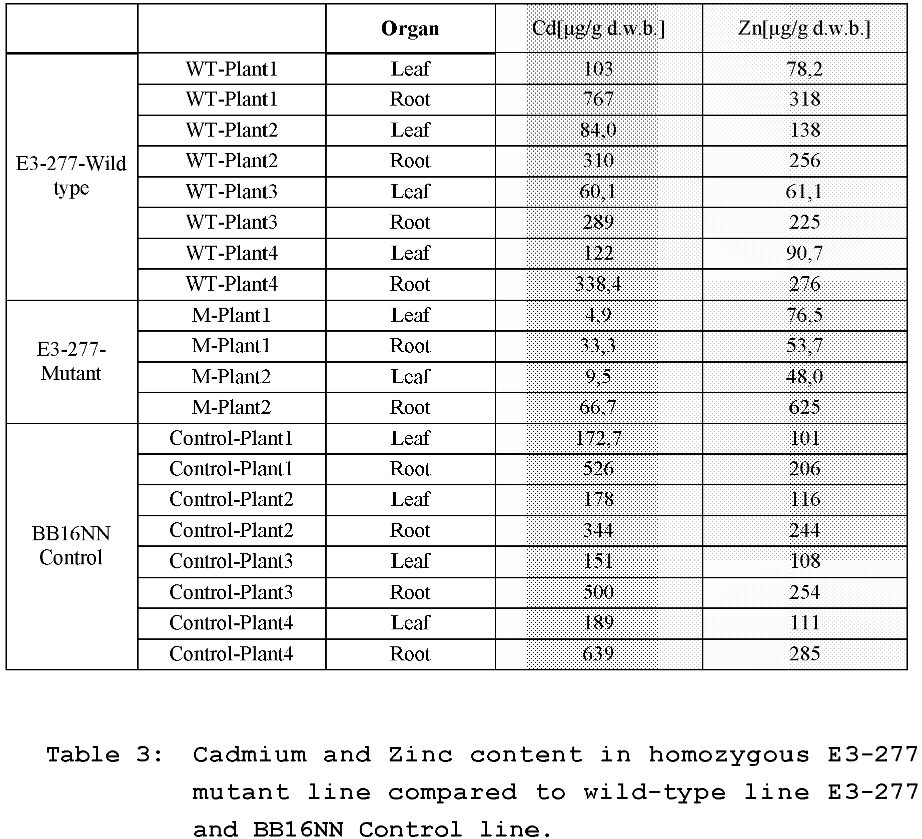

### EXAMPLE 7

In this example cadmium uptake of shoots of *N. tabacum* plants was tested. These shoots are from EMS treated plant lines mutated either in the HMA 4 gene derived from *N. sylvestris* or in the HMA 4 gene derived from *N. tomentosiformis.*

Cadmium uptake of shoots of transgenic *N. tabacum* plants was also tested. These transgenic plant lines express an RNAi construct designed to silence either one or both of the two HMA 4 genes.

### MATERIALS & METHODS

### EMS lines

All EMS lines analyzed in this example are summarized in Figure 13. Among the tested lines, 3 are mutated in the HMA 4 gene derived from *N. sylvestris* (identified as alpha gene in Figure 13) and 2 carry mutations in the HMA 4 gene derived from *N. tomentosiformis* (identified as beta gene in Figure 13).

The lines 90 and 425 were backcrossed 2 times. Backcrossing reduced the amount of additional mutations by 75%.

The line 277 was backcrossed once but the mutation is at the heterozygous state for this line. All the other lines carry homozygous mutations. The other lines were not backcrossed.

All lines were grown in the presence of cadmium using the protocol described above, except that in this Example the cadmium concentration was 1µM instead of 10µM. Each line was cultivated in a bowl that also contain the corresponding wild type plant, i.e. a plant that carries the same set of additional mutations in its genome but that lacks the mutation in the corresponding *HMA* gene.

### Transgenic lines

### 1) RNAi constructs

Five amiRNA constructs were made following the method described in Ossowski *et al,* 2008. An online resource (http://www.weigelworld.org/) was used to select the targeted sequences and design the corresponding primers. The result is presented in the Figure 14.

The amiRNAs were subcloned in a vector containing the "70S promoter" (35S promoter in which some enhancing regions are repeated) and a terminator (rbos). The promoter-amiRNA-terminator construct was inserted into a binary vector (pGreen) that permits expression *in planta.*

### 2) Hairpin constructs

The hairpin construct is designed to silence both of the *HMA* 4 genes. The targeted sequences as well as the primers used to amplify the same are described in Figure 15. The sequences were cloned into the pHannibal vector (Figure 16). pHannibal contains the appropriate restriction sites to clone the targeted sequences in both sense and antisense orientation.

The hairpin construct obtained was then subcloned twice and inserted into the pGreen vector with the same promoter and terminator used for the amiRNA constructs.

### 3) Plant transformation

Plants were transformed with the five amiRNA constructs, the hairpin construct and the empty pGreen vector as described in Horsch *et al,* 1984.

### 4) Growth conditions

The transgenic RNAi lines tested are the offspring of the plants regenerated after plant transformation.

RNAi lines were cultivated on the same Hoagland-derived media used for hydroponic culture but supplemented with 1% agar. The plants that lost their construct through segregation were eliminated by the addition of 200mg of hygromycin per liter of media. After two weeks of growth on selection media, plants were transferred to a media containing cadmium at a concentration of 1µM.

After two weeks of culture on the media containing cadmium, the plant material was collected. Approximately 1g of root was frozen in liquid nitrogen to analyze transcript accumulation by qPCR. The shoots were set aside to be analyzed for metal content by ICP-MS.

### 5) RNA extraction and cDNA synthesis

The roots were ground using a mortar and a pestle. RNA was obtained using the RNeasy kit (Qiagen) coupled with the DNase optional step. cDNA synthesis was performed with the M-MLV Reverse transcriptase (Promega) as described by the manufacturer.

### 6) Q-PCR analysis

The analyses were performed using a Roche 480 Lightcycler (Roche). The primer pairs used to amplify the alpha gene (Fw - ACAAAGTGCTCGGACACCAA ; Rev - CTTCTCGGTTGCAGAGTCCT) or the beta gene (Fw - ACAAAGTGCTCGGACACCAA ; Rev - CTTCTCGGTTGCAGAGTCTA) were designed to amplify specifically the targeted gene and not its homolog. This specificity and the efficiency of the primers were tested using a vector in which the region targeted by the primers was cloned.

The results were normalized using the cyclophilin gene (Fw - CTCTATGCCGACACCGTTCC ; Rev - TCACACGGTGGAAGGTTGAG) and the L2 gene (Fw - GGCGAAATGGGTCGTTTGATC ; Rev - CGTTCCGTTCGCCGAAGTCG). Those two reference genes were selected from the genes described in Nicot et al, 2005.

### RESULTS

### EMS lines

In 3 of the 5 tested lines, accumulation of cadmium in the shoots is reduced very significantly, namely by more than 50% (Figure 8 shows selected examples). These results were statistically validated for the lines 277 and 425 using the student t-test (Figure 9).

### RNAi lines

### 1. Transcript accumulation in roots

The transcript accumulation measured by qPCR is presented in Figure 10. No diminution in the transcript level was observed for the hairpin line as compared to the line expressing the empty vector.

However, a diminution of accumulation of HMA 4 transcripts is observed for both genes in line 6a and for the HMA 4 gene derived from *N. tomentosiformis* in line 11b. The expression levels are in a different static group than the expression level of the wild type plants.

### 2. Metal accumulation in shoots

The accumulation of cadmium is reduced in all analyzed transgenic lines (Figure 11). It is reduced by more than 40% in lines 6b and hairpin. It is reduced by more than 60% in lines 6a and 11b. A student's t-test performed on those samples showed that the difference observed in lines 6a and 11b in comparison to the wild type plants is significant. No statistical differences between the different lines in terms of cadmium accumulation and zinc accumulation were determined.

### CONCLUSION

This Example demonstrates that both *HMA* genes play a role in cadmium accumulation in shoots. Significant reduction of cadmium accumulation can already be achieved by silencing one of the *HMA* 4 genes. Further reduction of the cadmium accumulation can be obtained by silencing both genes.

### EXAMPLE 8

In this Example the Cadmium accumulation in shoots of several plants was analyzed and compared. For this purpose, the lines 90, 416, 276 and 425 were backcrossed 2 times and fixed by two self-pollinations to obtain BC2S2 plants, selected for the mutation (M) or not (W). The plants identified in this Example as wild-type plants (or W) are thus plants that have also been subject to EMS treatment and carry the same set of additional mutations in their genome but lack the mutation in the corresponding HMA gene.

Seeds were first sterilized and sown directly on solid medium in vitro. Lines were grown on Hoagland-derived medium with agar, containing 1µM of cadmium. The Hoagland-derived medium contains 0.1mM Fe and 15µM Zn.

Mutant lines (M), a heterozygous mutant line (Htz) and the corresponding wild-type (S) lines were analyzed. The experiments also included control plants BB16NN or BY02 (C) (wild-type, industrial seeds) and a tobacco RNAi line (obtained as described in Example 7).

Leaves were collected after one month and extracted for metals as previously described. Analyses were performed on 4 to 12 plants per genotype for Cd, Zn, Fe.

### RESULTS

The results are shown in Figures 17 and 18 and confirm that these plants have a significantly reduced amount of cadmium in the shoots (Figure 17B). At the same time it could be shown that the plants are still able to take up metals such as Fe and Zn that are required for plant growth (Figure 18).

### CONCLUSION

This Example demonstrates that the silencing of *HMA* genes in accordance with the present invention does not necessarily have a negative affect on the uptake of metals that are required for plant growth and development.

### EXAMPLE 9

To develop commercial tobacco varieties with reduced cadmium content in leaf, crosses were performed between different tobacco mutants. In particular, plants with a mutation in the HMA 4 gene from *N. sylvestris* were crossed with plants with a mutation in the HMA 4 gene from *N. tomentosiformis.*

For this purpose both mutants were backcrossed with the elite lines to eliminate the mutation load in the rest of the genome, which may cause problems with fertility in the progeny. The resulting BC1 plants were then crossed to get the F1 family, possessing two mutated copies at heterozygous state. All the F1 crosses are described in Figure 12.

The F1 plants resulting from the cross between mutants will be self-pollinated to obtain an F2 generation, in which homozygous plants for both mutated/or wild type copies will be present, including homozygous double mutant plants. These plants can be backcrossed into elite lines to obtain homozygous double mutant commercial plant lines.

### REFERENCES

Lugon-Moulin N, Martin F, Marc R. Krauss. Ramey PB, Rossi L (2006) Cadmium concentration in tobacco (Nicotiana tabacum L.) from different countries and its relationship with other elements. Chemosphere Vol 63 1074-1086.
Karaivazoglou NA, Tsotsolis NC and Tsadilas CD (2007) Influence of liming and form of nitrogen fertilizer on nutrient uptake, growth, yield, and quality of Virginia (flue-cured) tobacco. Field Crops Research Vol 100, Issue 1:52-60.
Verbruggen N, Hermans C, and Schat H (2009) Mechanisms to cope with arsenic or cadmium excess in plants. Current Opinion in Plant Biology 12:364-372.
Yeargan R, Maiti IB, Nielsen MT, Hunt AG, Wagner GJ (1992) Tissue partitioning of cadmium in transgenic tobacco seedlings and field grown plants expressing the mouse metallothionein I gene. Transgenic Res. Nov 1 (6):261-7.
Dorlhac De Borne F, Elmayan T, De Roton C, De Hys L, Tepfer M (1998) Cadmium partitioning in transgenic tobacco plants expressing a mammalian metallothionein gene. Molecular Breeding 4 :83-90.
Korenkov V, King B, Hirschi K, Wagner GJ (2009) Root-selective expression of AtCAX4 and AtCAX2 results in reduced lamina cadmium in field-grown Nicotiana tabacum L. Plant Biotechnol J. 7 (3):219-26.
Cobbett CS, Hussain D, Haydon MJ (2003) Structural and functional relationships between type 1B heavy metal-transporting P-type ATPases in Arabidopsis. New Phytologist 159:315-321.
Seigneurin-Berny D, Gravot A, Auroy P, Mazard C, Kraut A, Finazzi G, Grunwald D, Rappaport F, Vavasseur A, Joyard J, Richaud P, Rolland N (2006) HMA1, a new Cu-ATPase of the chloroplast envelope, is essential for growth under adverse light conditions. The journal of biological chemistral 281:2882-2892.
Hussain D, Haydon M, Wang Y, Wong E, Sherson S, Young J, Camakaris J, Harper J, Cobbett C (2004) P-Type ATPase heavy metal transporters with roles in essential zinc homeostasis in Arabidopsis. The Plant Cell 16 :1327-1339.
Morel M, Crouzet J, Gravot A, Auroy P, Leonhardt N, Vavasseur A, Richaud P (2009) AtHMA3, a P1B-ATPase Allowing Cd/Zn/Co/Pb Vacuolar Storage in Arabidopsis. Plant physiology 149:894-904.
HAYES, Alec et al., TRANSGENIC PLANTS MODIFIED FOR REDUCED CADMIUM TRANSPORT, DERIVATIVE PRODUCTS, AND RELATED METHODS, WO2009/074325.
Julio E, Laporte F, Reis S, Rothan C and Dorlhac de Borne F (2008) Reducing the content of nornicotine in tobacco via targeted mutation breeding. Molecular Breeding, 21:1380-3743.
Yukawa M, Tsudzuki T, Sugiura M (2006) The chloroplast genome of Nicotiana sylvestris and Nicotiana tomentosiformis: complete sequencing confirms that the Nicotiana sylvestris progenitor is the maternal genome donor of Nicotiana tabacum. Molecular genetics and genomics 275:367-373.
Delon R, Poisson C, Bardon JC, Taillurat P (1999) Les nicotianées en collection à l'Institut du Tabac. SEITA, 86p.
Till BJ, Reynolds SH, Greene EA et al (2003) Large-scale discovery of induced point mutations with high-throughput TILLING. Genome Res. 13:524-530.
Ng PC and Henikoff S (2003) SIFT: predicting amino-acid changes that affect protein function. Nucleic Acid Res. 31:3812-3814.
Newman, Alan. 1996. "Elements of ICPMS." Analytical Chemistry. 68(Jan 1):46A-51A.
Kelly S, Baxter M, Chapman S, Rhodes C, Dennis J, and Brereton P (2002) The application of isotopic and elemental analysis to determine the geographical origin of premium long grain rice. European food research & technology, 214:72-82.
Ossowski et al., 2008, "Gene silencing in plants using artificial microRNAs and other small RNAs" The Plant Journal, February 2008, vol. 53(4): 674-690.
Horsch et al., 1984, "Inheritance of functional foreign genes in plants" Science 1984 Feb 3; 223(4635):496-8.
Nicot et al., "Housekeeping gene selection for real-time RT-PCR normalization in potato during biotic and abiotic stress", J Exp Bot. 2005 Nov; 56(421):2907-14.

## Claims

1. A tobacco plant comprising at least one mutation in a HMA gene, wherein the non-mutated HMA gene comprises the nucleotide sequence of SEQ ID NO:1 or a homolog sequence with at least 90% identity to the sequence of SEQ ID NO:1, wherein the mutation causes a substitution or a deletion or an insertion of at least one amino acid in the polypeptide encoded by the nucleotide sequence and wherein the mutation reduces the heavy metal uptake by the leaves of the plant by at least 30% in relation to the heavy metal uptake of plants comprising SEQ ID NO:1 or the homolog.

2. The tobacco plant according to claim 1, wherein the tobacco plant is a *Nicotiana tabacum* plant.

3. The tobacco plant according to claim 1 or 2, wherein the reduction of heavy metal uptake is determined by growing tobacco plants with and without the mutation under identical conditions on a liquid medium containing the heavy metal and comparing the concentration of the heavy metal in the leaf, stem or shoot of the tobacco plant with the mutation to the concentration of the heavy metal in the leaf, stem or shoot of the tobacco plant that does not have the mutation.

4. The tobacco plant according to one of claims 1 to 3, wherein the mutation reduces the uptake of the heavy metal by at least 40%, at least 50%, at least 75% or at least 95%.

5. The tobacco plant according to one of claims 1 to 4, wherein the mutation reduces the uptake of cadmium, lead or arsenic.

6. The tobacco plant according to one of claims 1 to 5, wherein the mutation is a miss-sense, a non-sense mutation or a splice mutation.

7. The tobacco plant according to claim 6, wherein the mutation is selected from the group comprising the mutations: G294A, C576T, G406A, G347A, G363A, G553A, G290A, C374T, G964A, G1168A, G1211A, G1126A, C980T, G1195T, G1156A, G1070A, C2302T, G2208A, C2217T, G2190A, C2206T or C2277T in SEQ ID NO:1 or the homolog.

8. The tobacco plant according to one of claims 1 to 7, wherein the plant comprises a mutation in more than one HMA 4 gene.

9. The tobacco plant according to one of claims 1 to 8, wherein the plant is homozygous for the mutation in the HMA 4 gene.

10. The tobacco plant according to one of claims 1 to 9, wherein the plant is homozygous for mutations in both HMA 4 genes.

11. Part of a *tobacco* plant according to one of claims 1 to 10, wherein the part is a leaf, a lamina, a cut and/or a cured leaf, root, shoot, stem, flower or seed and wherein the part comprises at least one mutation in a HMA gene, wherein the non-mutated HMA gene comprises the nucleotide sequence of SEQ ID NO:1 or a homolog sequence with at least 90% identity to the sequence of SEQ ID NO:1, wherein the mutation causes a substitution or a deletion or an insertion of at least one amino acid in the polypeptide encoded by the nucleotide sequence and wherein the mutation reduces the heavy metal uptake by the leaves of the plant by at least 30% in relation to the heavy metal uptake of plants comprising SEQ ID NO:1 or the homolog.

12. The part of a *tobacco* plant according to claim 11, wherein the tobacco plant is a *Nicotiana tabacum* plant.

13. Tobacco product comprising a tobacco plant according to one of claims 1 to 10 or a part of a tobacco plant according to claim 11 or 12.

14. Tobacco product according to claim 13, wherein the tobacco product is a cut tobacco, tobacco extract or reconstituted tobacco or a smokeless tobacco product, like snus or snuff.

15. Smoking article comprising tobacco plants according to one of claims 1 to 10 or parts of a tobacco plant according to claims 11 to 12.

16. Smoking article according to claim 15, which is a cigarette, a small cigar, cigarillo, a cigar or a simulated smoking article containing tobacco.

## Patentansprüche

1. Tabakpflanze, die mindestens eine Mutation in einem HMA Gen aufweist, wobei das nicht-mutierte HMA Gen die Nukleotidsequenz der SEQ ID Nr.: 1 oder eine homologe Sequenz mit mindestens 90% Identität zu der Sequenz der SEQ ID Nr.: 1 aufweist, wobei die Mutation eine Substitution oder eine Deletion oder eine Insertion von mindestens einer Aminosäure in dem Polypeptid bewirkt, welches von der Nukleotidsequenz kodiert wird, und wobei die Mutation die Aufnahme eines Schwermetalls in den Blättern der Pflanze um mindestens 30% im Vergleich zur Aufnahme eines Schwermetalls durch Pflanzen verringert, welche SEQ ID Nr.: 1 oder dessen Homolog aufweisen.

2. Tabakpflanze gemäß Anspruch 1, wobei die Tabakpflanze eine *Nicotiana tabacum* Pflanze ist.

3. Tabakpflanze gemäß Anspruch 1 oder 2, wobei die Verringerung der Aufnahme eines Schwermetalls nachgewiesen wird, indem man Tabakpflanzen mit und ohne der Mutation unter identischen Bedingungen in einem Flüssigmedium zieht, welches das Schwermetall enthält, und die Konzentration des Schwermetalls im Blatt, Stamm oder Spross der Tabakpflanze mit der Mutation mit der Konzentration des Schwermetalls im Blatt, Stamm oder Spross der Tabakpflanze ohne die Mutation vergleicht.

4. Tabakpflanze gemäß einem der Ansprüche 1-3, bei dem die Mutation die Aufnahme des Schwermetalls um mindestens 40%, mindestens 50%, mindestens 75% oder mindestens 95% verringert.

5. Tabakpflanze gemäß einem der Ansprüche 1-4, wobei die Mutation die Aufnahme von Kadmium, Blei oder Arsen verringert.

6. Tabakpflanze gemäß irgendeinem der Ansprüche 1-5, wobei die Mutation eine Missense, Nonsense Mutation oder Splice-Mutation ist.

7. Tabakpflanze gemäß Anspruch 6, wobei die Mutation aus der Gruppe bestehend aus den Mutationen G294A, C576T, G406A, G347A, G363A, G553A, G290A, C374T, G964A, G1168A, G1211A, G1126A, C980T, G1195T, G1156A, G1070A, C2302T, G2208A, C2217T, G2190A, C2206T or C2277T der SEQ ID Nr.: 1 oder des Homologs ausgewählt wurde.

8. Tabakpflanze gemäß irgendeinem der Ansprüche 1-7, wobei die Pflanze eine Mutation in mehr als einem HMA 4 Gen umfasst.

9. Tabakpflanze gemäß irgendeinem der Ansprüche 1-8, wobei die Pflanze für die Mutation in dem HMA 4 Gen homozygot ist.

10. Tabakpflanze gemäß irgendeinem der Ansprüche 1-9, wobei die Pflanze für Mutationen in beiden HMA 4 Genen homozygot ist.

11. Teil der Tabakpflanze gemäß irgendeinem der Ansprüche 1-10, wobei der Teil ein Blatt, eine Blattspreite, ein geschnittenes und/oder behandeltes Blatt, Wurzel, Stammspross, Stamm, Blüte oder Samen ist und wobei das Teil mindestens eine Mutation in einem HMA Gen aufweist, wobei das nicht-mutierte HMA Gen die Nukleotidsequenz der SEQ ID Nr.: 1 oder einer homologen Sequenz mit mindestens 90% Identität zur Sequenz der SEQ ID Nr.: 1 aufweist, wobei die Mutation eine Substitution oder eine Deletion oder eine Insertion von mindestens einer Aminosäure in dem Polypeptid verursacht, dass durch die Nukleotidsequenz kodiert wird, und wobei die Mutation die Aufnahme eines Schwermetalls durch die Blätter der Pflanze um mindestens 30% im Vergleich zur Aufnahme des Schwermetalls durch Pflanzen verringert, welche SEQ ID Nr.: 1 oder dessen Homolog umfassen.

12. Teil einer Tabakpflanze gemäß Anspruch 11, wobei die Tabakpflanze eine *Nicotiana tabacum* Pflanze ist.

13. Tabakprodukt, das eine Tabakpflanze gemäß einem der Ansprüche 1-10 oder einem Teil einer Tabakpflanze gemäß Anspruch 11 oder 12 umfasst.

14. Tabakprodukt gemäß Anspruch 13, wobei das Tabakprodukt ein geschnittener Tabak, Tabakextrakt oder rekonstituierter Tabak oder ein rauchfreies Tabakprodukt, wie Snus oder Snuff ist.

15. Rauchartikel, der eine Tabakpflanze gemäß irgendeinem der Ansprüche 1-10 oder Teile einer Tabakpflanze gemäß Anspruch 11 oder 12 umfasst.

16. Rauchartikel gemäß Anspruch 15, bei dem es sich um eine Cigarette, ein kleine Cigarre, einen Cigarillo, eine Cigarre oder einen Tabak enthaltenden simulierten Rauchartikel handelt.

## Revendications

1. Plant de tabac portant au moins une mutation dans un gène HMA, lequel gène HMA, à l'état non-muté, comprend la séquence de nucléotides de la Séquence N° 1 ou une séquence homologue, identique, à un degré d'au moins 90 %, à la séquence de la Séquence N° 1, laquelle mutation provoque le remplacement, la délétion ou l'insertion d'au moins un acide aminé dans le polypeptide codé par la séquence de nucléotides, et laquelle mutation réduit d'au moins 30 % l'absorption d'un métal lourd par les feuilles du plant, par rapport à l'absorption du métal lourd par des plants comportant la Séquence N° 1 ou son homologue.

2. Plant de tabac conforme à la revendication 1, lequel plant de tabac est un plant de *Nicotiana tabacum*.

3. Plant de tabac conforme à la revendication 1 ou 2, chez lequel on détermine la réduction de l'absorption d'un métal lourd en faisant pousser, dans des conditions identiques et sur un milieu liquide contenant le métal lourd, des plants de tabac portant la mutation et d'autres qui ne la portent pas, et en comparant la concentration du métal lourd dans les feuilles, la tige ou les pousses d'un plant de tabac porteur de la mutation avec la concentration du métal lourd dans les feuilles, la tige ou les pousses d'un plant de tabac non porteur de la mutation.

4. Plant de tabac conforme à l'une des revendications 1 à 3, chez lequel la mutation réduit l'absorption du métal lourd d'au moins 40 %, d'au moins 50 %, d'au moins 75 %, ou d'au moins 95 %.

5. Plant de tabac conforme à l'une des revendications 1 à 4, chez lequel la mutation réduit l'absorption du cadmium, du plomb ou de l'arsenic.

6. Plant de tabac conforme à l'une des revendications 1 à 5, chez lequel la mutation est une mutation faux-sens, une mutation non-sens ou une mutation d'épissage.

7. Plant de tabac conforme à la revendication 6, chez lequel la mutation est choisie dans l'ensemble constitué par les mutations suivantes : G294A, C576T, G406A, G347A, G363A, G553A, G290A, C374T, G964A, G1168A, G1211A, G1126A, C980T, G1195T, G1156A, G1070A, C2302A, G2208A, C2217T, G2190A, C2206T et C2277T, dans la Séquence N° 1 ou son homologue.

8. Plant de tabac conforme à l'une des revendications 1 à 7, lequel plant porte une mutation dans plus d'un gène HMA4.

9. Plant de tabac conforme à l'une des revendications 1 à 8, lequel plant est homozygote pour la mutation dans le gène HMA4.

10. Plant de tabac conforme à l'une des revendications 1 à 9, lequel plant est homozygote pour les mutations dans les deux gènes HMA4.

11. Partie d'un plant de tabac conforme à l'une des revendications 1 à 10, laquelle partie est une feuille, un limbe, ou une feuille, racine, pousse, tige, fleur ou graine à l'état coupé et/ou séché, et laquelle partie porte au moins une mutation dans un gène HMA, lequel gène HMA, à l'état non-muté, comprend la séquence de nucléotides de la Séquence N° 1 ou une séquence homologue, identique, à un degré d'au moins 90 %, à la séquence de la Séquence N° 1, laquelle mutation provoque le remplacement, la délétion ou l'insertion d'au moins un acide aminé dans le polypeptide codé par la séquence de nucléotides, et laquelle mutation réduit d'au moins 30 % l'absorption d'un métal lourd par les feuilles du plant, par rapport à l'absorption du métal lourd par des plants comportant la Séquence N° 1 ou son homologue.

12. Partie d'un plant de tabac, conforme à la revendication 11, pour laquelle le plant de tabac est un plant de *Nicotiana tabacum*.

13. Produit à base de tabac, provenant d'un plant de tabac conforme à l'une des revendications 1 à 10 ou d'une partie de plant de tabac conforme à la revendication 11 ou 12.

14. Produit à base de tabac conforme à la revendication 13, lequel produit à base de tabac est un tabac coupé, un extrait de tabac ou un tabac reconstitué, ou un produit à base de tabac qui n'est pas à fumer, comme du tabac à priser ou du tabac à chiquer de type snus.

15. Article à fumer, comprenant du tabac provenant d'un plant conforme à l'une des revendications 1 à 10 ou de parties de plant de tabac conformes à la revendication 11 ou 12.

16. Article à fumer conforme à la revendication 15, qui est une cigarette, un petit cigare, un cigarillo, un cigare ou un simulacre d'article à fumer contenant du tabac.
